# EUROPEAN PATENT APPLICATION

(11) **EP 4 420 680 A1**
(43) Date of publication of application: **28.08.2024**
(21) Application number: 22882177.3
(22) Date of filing: 14.01.2022
(51) Int. Cl.: A61K 47/54, A61K 47/22, A61K 38/28, A61K 9/00, A61K 45/06, A61K 9/51, A61K 47/69, A61P 3/10, B82Y 5/00, B82Y 40/00

(54) **AGGREGATE FORMED BY MEANS OF ASSEMBLING CHALCOGEN HETEROCYCLIC COMPOUND AND INSULIN, AND PREPARATION METHOD THEREFOR, AND INSULIN ORAL PREPARATION**

(30) Priority: 21.10.2021 CN 202111228794
(71) Applicant: Fuzhou University, Fuzhou, Fujian 350108 (CN)
(72) Inventor: CHEN, Zhaowei, Fuzhou, Fujian 350108 (CN); CHEN, Chen, Fuzhou, Fujian 350108 (CN); YANG, Huanghao, Fuzhou, Fujian 350108 (CN); LIU, Zheng, Fuzhou, Fujian 350108 (CN); HE, Yu, Fuzhou, Fujian 350108 (CN)
(74) Representative: Prinz & Partner mbB
(86) International application number: PCT/CN2022/072018
(87) International publication number: WO 2023/065551

(57) **Abstract**

Provided in the present invention are an aggregate and a preparation method therefor, and an insulin oral preparation. Specifically provided is an aggregate, which is an aggregate formed by means of assembling a chalcogen heterocyclic compound and insulin. The aggregate can be further prepared into an insulin oral preparation. The oral preparation can be used for reducing the blood glucose level of a mammal as part of a diabetes treatment regimen. The chalcogen heterocyclic compound in the insulin oral preparation prepared by means of the method can effectively protect insulin against the three physiological barriers of an oral uptake pathway, is stable in the gastrointestinal tract environment, is subjected to a dynamic chemical exchange reaction with intestinal mucin in intestinal juice and the sulfhydryl groups of proteins inside and outside an epithelial cell membrane by means of the molecular bond of polychalcogen on the surface, and enters the circulation system from the intestinal epithelial cell to achieve the effect of reducing blood glucose. When being orally administered, the insulin oral preparation has high bioavailability, has a good hypoglycemic effect in mammals, and can be used for treating diabetes.

## Description

This application claims the priority of Chinese Patent Application No. 202111228794.3, filed with the China National Intellectual Property Administration on 21 October 2021, and titled with "AGGREGATE FORMED BY MEANS OF ASSEMBLING CHALCOGEN HETEROCYCLIC COMPOUND AND INSULIN, AND PREPARATION METHOD THEREFOR, AND INSULIN ORAL FORMULATION", the disclosure of which is hereby incorporated by reference in its entirety.

### FIELD

The present invention relates to the field of biomedical technology, and in particular relates to an aggregate formed by assembling a chalcogen heterocyclic compound and insulin, a preparation method thereof and an oral formulation of insulin.

### BACKGROUND

Diabetes mellitus is a common chronic disease seriously threatening human life and health. According to the data in the IDF Diabetes Atlas 9th Edition published by the International Diabetes Federation, it shows that by 2019, 463 million people worldwide suffer from diabetes mellitus. With the improvement of life quality resulting in a change in dietary habit and lifestyle, the prevalence of diabetes mellitus is increasing year by year, and this number is predicted to rise to 700 million by 2045. Diabetes mellitus is mainly characterized by high blood glucose in clinical, and persistent high blood glucose and long-term metabolic disorder will lead to various complications, such as cardiovascular diseases, peripheral vascular diseases, nervous system diseases, retinopathy, kidney disease and kidney failure, ulcers and amputations, metabolic complications, oral complications, and depression, ultimately leading to loss of labor capacity, even death and disability.

Insulin is the sole hormone responsible for lowering blood glucose in the body, and it has been used in the treatment of diabetes for over a century. Insulin is not only the drug of choice for the treatment of insulin-dependent type I diabetes mellitus, but also an adjunctive drug in the treatment of mid- and late-stage type II diabetes mellitus. Data from clinical studies have shown that, intensive insulin therapy for patients newly diagnosed with early-stage type II diabetes mellitus can achieve a 2-year clinical remission in about half of these patients, suggesting that insulin is important in the treatment of both type I and type II diabetes. Insulin has been since its inception mainly administered by subcutaneous or intravenous injection for the treatment of diabetes mellitus, at 2-4 injections or more a day. For patients with diabetes who require long-term insulin medication, frequent injections may cause great pain and inconvenience to the patient. Moreover, the difficulty in controlling the amount injected leads to large fluctuations in blood glucose levels easily, and long-term injections lead to poor patient compliance and adverse reactions such as tissue necrosis at the injection site, microbial infection, insulinemia, and nerve damage. In addition, because the physiological delivery pathway of the ingested insulin by injection is different from that of the insulin secreted by the pancreas, the liver does not obtain a necessary concentration of insulin to control blood glucose, resulting in hyperinsulinemia. Therefore, the study and development of an insulin formulation that is easy to administer, safe and reliable for non-injectable administration is of great clinical importance for improving the health and life quality of diabetic patients, and is also an important issue of general concern to the domestic and international pharmaceutical industry.

For non-injectable administration, oral administration is the most traditional, convenient, and patient-acceptable manner. An oral formulation has low manufacturing requirements, and is stable in storage, easy to carry and uptake, and relatively low at cost. Moreover, the insulin ingested orally can better imitate the insulin secreted by pancreas in pharmacokinetics as they both enter the liver via the portal vein and are then delivered to the peripheral circulation. However, the insulin belongs to a polypeptide drug, and the oral formulation of insulin, which is ingested orally and enters the circulatory system to lower blood glucose, needs to pass through three physiological barriers as follows: (1) The strong acidic environment in the stomach and digestive enzymes such as pepsin, trypsin, and peptidases presented in the gastrointestinal tract readily denature and degrade insulin, resulting in loss of biological activity of insulin. (2) A dense net-like structure formed by disulfide bonding or non-covalent interaction of mucoproteins in the mucus layer throughout the gastrointestinal tract rejects and hinders the diffusion and penetration of the insulin formulation, making it difficult to approach the intestinal epithelial cells, and restricting the absorption of insulin. (3) The insulin has a low absorption rate by intestinal epithelial cells due to its large molecular weight compared to a small molecule drug. The intercellular junction proteins between the intestinal epithelial cells and intracellular degradation by lysosome prevent insulin from entering the circulatory system through the paracellular or transcellular pathway, thus restricting its bioavailability.

Currently, various encapsulating methods to prepare an oral formulation of insulin have been reported to overcome these barriers and improve the bioavailability of insulin, but a number of problems still remain. Firstly, the existing methods for preparing an oral formulation of insulin are all very complex, requiring a large amount of chemical synthesis or physical processes for the modification of insulin, the preparation of the encapsulating agent, and the encapsulating of insulin. Secondly, in addition to the encapsulating agent and insulin, it is necessary to add one or more additives such as an absorption enhancer, a stabilizer, an enzyme inhibitor, a permeation enhancer, a pH adjusting agent, a dissolving agent for mucoproteins or adhesive agent, and a cell-permeable peptide to overcome the three physiological barriers above. These additives can easily cause damage to the normal physiological functions of the gastrointestinal tract and infections, and a large amount of systematic clinical research is still desirable for the biosafety issue. Again, the fact that a formulation contains multiple functional components increases the cost of an oral formulation of insulin, and also leads to a low efficiency of encapsulating of insulin. Taken together, these factors limit the popular use of oral formulations of insulin in clinical. Therefore, there is an urgent need to develop a simple and effective insulin oral formulation that can overcome the three physiological barriers when it is ingested orally, to reduce the cost of preparation, improve the pharmacokinetic and pharmacodynamic characteristics of insulin ingested orally, enhance the bioavailability of insulin ingested orally, and improve the effect of lowering blood glucose by insulin ingested orally.

### SUMMARY

In view of this, the technical problem to be solved by the present invention is to provide an aggregate formed by assembling a chalcogen heterocyclic compound and insulin, a preparation method thereof, and an oral formulation of insulin. The oral formulation of insulin has a high bioavailability when administered orally.

The present invention provides an aggregate formed by assembling a chalcogen heterocyclic compound and insulin;

wherein the chalcogen heterocyclic compound has a structure represented by formula I,

A-L-B Formula I;

wherein, A is a heterocyclic group containing two or more same and/or different atoms of chalcogen selected from the group consisting of sulfur, selenium and tellurium;
B is a group interacting with an insulin molecule, and
L is a linker linking A and B.

In the present invention, the heterocyclic group A comprises only one chalcogen or any combination of the three chalcogen above.

The heterocyclic group A is preferably a 4-100 membered ring.

In the present invention, preferably, A has a structure selected from the group consisting of:

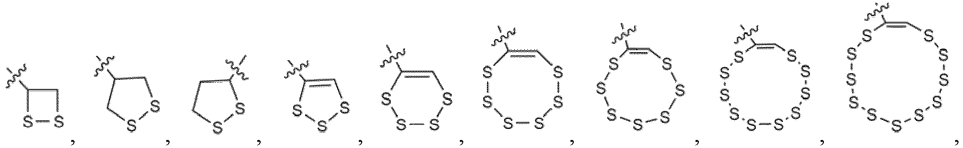

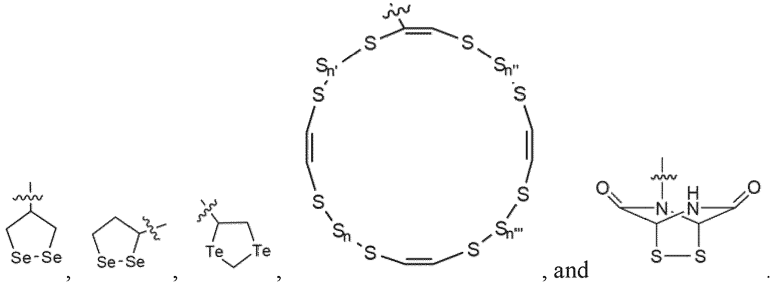

In the present invention, L is a linker linking functional group A and functional group B.

Preferably, L is selected from the group consisting of a carbon-carbon bond, a carbon-boron bond, a carbon-nitrogen bond, a carbon-phosphorus bond, a carbon-oxygen bond, a carbon-sulfur bond, a carbon-selenium bond, a carbon-tellurium bond, a metal-ligand bond, a boron-ester bond, a disulfide bond, a ring-forming group, a hydrogen bond, a cleavable chemical bond, a supramolecular host-guest interaction, and a ligand-receptor recognition interaction.

The linkers above are the binding of L to A or L to B. The binding of L to A, and the binding of L to B are the same or different.

In the present invention, the functional group B and an insulin molecule drive, through intermolecular interaction, the assembly of the compound represented by formula I with insulin into an aggregate. The intermolecular interaction is a covalent bond, a non-covalent bond or both covalent and non-covalent bonds. The covalent bond is selected from the group consisting of an amide bond, an ester bond, a ligand bond, a click chemical bond, an ether bond, an ester-amide bond, an imide bond, a boron-ester bond, a disulfide bond, a carbon-carbon single bond, a carbon-carbon double bond, a carbon-carbon triple bond, and a combination thereof. The non-covalent bond is selected from the group consisting of an electrostatic interaction, a van der Waals force, a hydrophobic interaction, a supramolecular host-guest interaction, a ligand-acceptor recognition interaction, a salt bridge and a combination thereof.

Preferably, B is selected from the group consisting of a chemical/biological molecule recognizing insulin, a DNA complementary strand, an aptamer, a polypeptide having a coiled coil/zipper structure/superstructure, and a chemical group selected from the group consisting of: 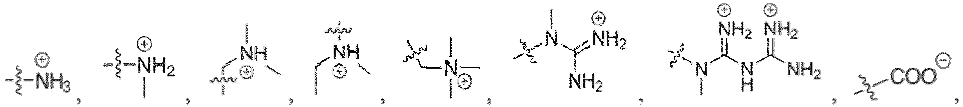 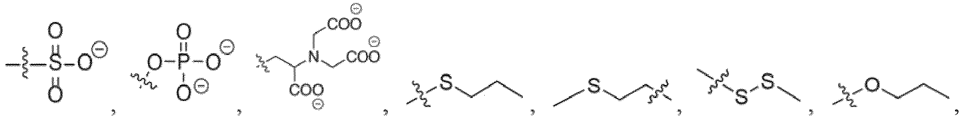 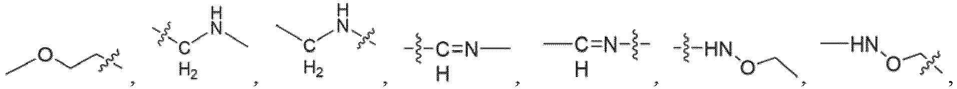 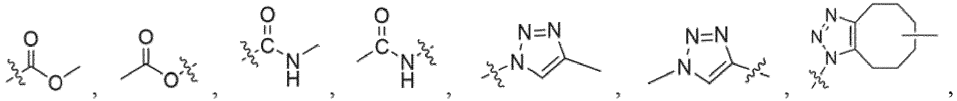 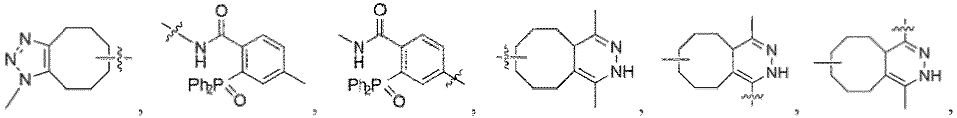 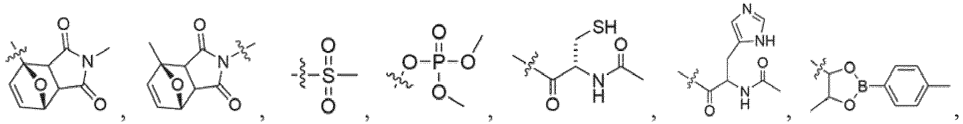 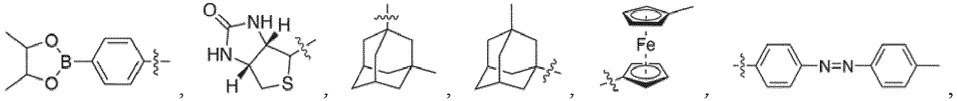 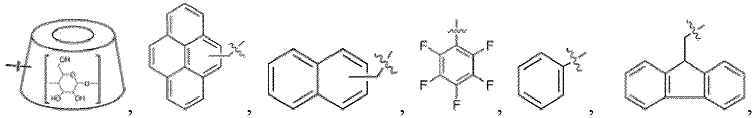 and a combination thereof.

Further preferably, the chalcogen heterocyclic compound has a structure represented by formula II:

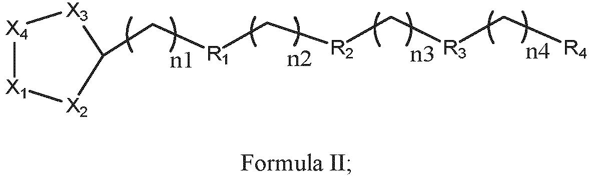

wherein, X₁, X₂, X₃, and X₄ are independently selected from the group consisting of S, Se, Te and C, and at least two of X₁, X₂, X₃, and X₄ are independently selected from the group consisting of S, Se and Te;

R₁, R₂, and R₃ are independently selected from the group consisting of a carbon atom, amido and imino;

R₄ is selected from carboxyl, amino, 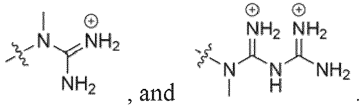 ; and

n1, n2, n3, and n4 are any integers independently selected from 1 to 6.

In the structure formula of the present invention, the curved line " 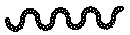 " represents the connection position; and the single bond represented by "-" denotes methyl.

For a single bond of a cyclic group or cyclic aryl group, for example, 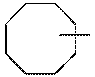 and 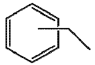 indicate that methyl or ethyl can be connected to the cyclic group or the cyclic aryl group at any position, respectively.

For a substituent of a cyclic group or cyclic aryl group, for example, 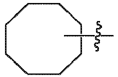 and 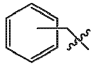 indicate that a substituent can be connected to the cyclic group or the cyclic aryl group at any position, respectively.

In some particular embodiments of the present invention, the chalcogen heterocyclic compound has a structure selected from the group consisting of 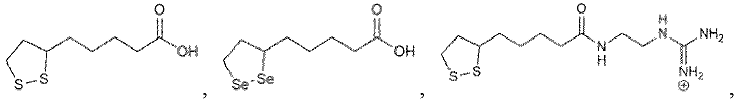 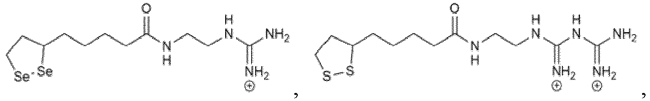 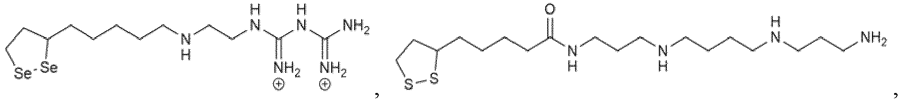 and a combination thereof.

In the present invention, the aggregate formed by assembling a chalcogen heterocyclic compound and insulin comprises one or more chalcogen heterocyclic compounds.

The present invention has no special limitation on the type of insulin, and the insulin is selected from the group consisting of unmodified insulin, an insulin analogue, modified insulin, and a combination thereof.

Preferably, the unmodified insulin is selected from the group consisting of human insulin, human recombinant insulin, bovine insulin, porcine insulin, semi-synthetic insulin, biosynthetic insulin, and a combination thereof.

Preferably, the insulin analogue is selected from the group consisting of insulin lispro, insulin glulisine, insulin aspart, insulin detemir, insulin glargine, insulin degludec; and a combination thereof.

The modified insulin is selected from the group consisting of chemically modified insulin, biologically modified insulin, genetically engineered insulin, and a combination thereof.

In the present invention, the insulin is selected from the group consisting of regular insulin, mealtime insulin, ultra-rapid-acting insulin, rapid-acting insulin, intermediate-acting insulin, long-acting insulin, premixed insulin, and a combination thereof.

Preferably, a molar ratio of the chalcogen heterocyclic compound to the insulin is 1 -200:1 in the present invention.

Preferably, the aggregate has a particle size of 10-500 nm in the present invention.

In the present invention, the aggregate of insulin and the chalcogen heterocyclic compound has high stability in simulated gastric and intestinal fluids, which avoids the degradation and leakage of insulin in an acidic environment and an environment with digestive enzymes.

The present invention provides a method for producing the aggregate, comprising
S1) incubating a solution of insulin with a solution of a chalcogen heterocyclic compound, assembling, and then obtaining an aggregate.

Specifically, S1 is performed by firstly incubating a solution of insulin with a solution of a chalcogen heterocyclic compound, assembling, then obtaining an aggregate as a colloidal particle, then removing free chalcogen heterocyclic compound and/or insulin which are not involved in the assembling, and finally obtaining a suspension of the aggregate assembled by insulin and chalcogen heterocyclic compound.

The incubating is performed for preferably 30 minutes.

Upon the above incubating, the chalcogen heterocyclic compound and insulin molecule can assemble, through intermolecular interactions, into an aggregate as a colloidal particle with a particle size of 10-500 nm, thereby achieving the encapsulation of insulin, with an encapsulation efficiency of up to 70%.

The present invention has no special limitations on the methods of removing free chalcogen heterocyclic compound and/or insulin which are not involved in the assembling, which may be a method well known to those skilled in the art, including but not limited to dialysis or centrifugation.

The method for producing the aggregate disclosed in the present invention has a simple operation and requires a mild condition. The oral formulation of insulin prepared by this method does not need additional functional additives, and the cost is low.

The interaction between the chalcogen heterocyclic compound and insulin during the formation of the aggregate causes a greatly increased local concentration, such that the heterocyclic groups are in close proximity to each another, causing a ring-opening polymerization, leading to formation of molecular bonds of polychalcogen, and allowing the chalcogen hetero-atomic compound to be presented in a form selected from the group consisting of a monomer, a short-chain oligomer, a long-chain polymer, and a combination thereof.

The present invention has no special limitations on the temperature, reaction duration, buffer solution and solvent used in the process of assembling the aggregate, which can be a condition known to those skilled in the art.

The present invention provides use of the aggregate in the manufacture of a medicament for lowering blood glucose.

In the present invention, an aqueous suspension of the aggregate of insulin and a chalcogen heterocyclic compound can be administered orally directly to a mammal.

Based on this, the present invention provides an oral formulation of insulin comprising the aggregate, and a pharmaceutically acceptable excipient.

The present invention does not specifically limit the dosage form of the oral formulation, which can be an oral dosage form known to those skilled in the art.

In the present invention, the aggregate of insulin and a chalcogen heterocyclic compound can be prepared into an enteric formulation for oral administration, such as an enteric-coated capsule, a powder, a tablet, a granule, a suspension, and a drop pill, using a pharmaceutically acceptable excipient. Preferably, a dry powder of the aggregate of insulin and a chalcogen heterocyclic compound is combined with a pharmaceutically acceptable enteric-coated capsule for oral administration.

The present invention has no special limitation on the excipient, and those skilled in the art can select according to the dosage form.

In the present invention, a dynamic chemical exchange reaction can occur between the molecular bond of polychalcogen on the surface of the aggregate of insulin and chalcogen heterocyclic compound and the sulfhydryl on the surface of mucoproteins and other glycoproteins, which promotes the rapid penetration of the aggregate through the intestinal mucus layer. The molecular bond of polychalcogen is selected form the group consisting of a polysulfur bond, a polyselenium bond, a polysellurium bond, and a molecular bond of polychalcogen containing more than one chalcogen.

In the present invention, the aggregate of insulin and chalcogen heterocyclic compound, without destroying the tight junction protein between the epithelial cells, enters the cytoplasm via a non-endocytosis transcellular pathway by causing a dynamic chemical exchange reaction between the polychalcogen on the surface of the aggregate and the sulfhydryl on the outer surface of an epithelial cell membrane, avoiding being degraded by a lysosome. Then the aggregate enters into circulation system via intestinal epithelial cells by causing a dynamic chemical exchange reaction with the sulfhydryl on the inner surface of an epithelial cell membrane. This process promotes the uptake of insulin by epithelial cells, thereby achieving a high bioavailability of insulin and a rapid effect of lowering blood glucose by insulin.

In the present invention, after entering the blood circulation, the aggregate assembled by insulin and chalcogen heterocyclic compound reaches the hepatic sinusoids via the portal circulation, and is depolymerized by glutathione in the hepatic sinusoids to release insulin to lower blood glucose. The aggregate is depolymerized by an exchange reaction between the glutathione and the molecular bond of polychalcogen, which results in the degradation of a short-chain oligomer or a long-chain polymer of the chalcogen heteroatomic compound of the aggregate into a monomer or shorter-chain oligomer, thus failing to form a stable aggregate.

The present invention provides a method for lowering blood glucose in a mammal. The method comprises administering orally to a mammal a suspension of the aggregate of insulin and chalcogen heterocyclic compound or administering orally to a mammal a composition comprising a dry powder (e.g., a lyophilized powder) of the aggregate of insulin and chalcogen heterocyclic compound and a pharmaceutically acceptable excipient, to lower blood glucose in mammals.

In the present invention, the oral insulin formulation is administered to a mammal, including but not limited to, a mouse, a rat, a rabbit, a dog, a cat, sheep, a pig, a cow, a horse, a monkey, and a human; preferably, the mammal is a human in need of lowering blood glucose, such as a patient with diabetes.

In the present invention, the lowering blood glucose in a mammal refers to, a reduction of at least 7%, preferably at least 30%, of the blood glucose of the mammal within 15 minutes after orally administering to the mammal a therapeutically effective amount of the aggregate of insulin and chalcogen heterocyclic compound. More preferably, the blood glucose of the mammal is reduced by at least 30%, preferably by at least 45%, more preferably by at least 75% over 15-840 minutes after administering to the mammal the aggregate.

In the present invention, there is no particular limitation on the amount of the insulin to be administered. The amount of insulin to be administered varies depending on the species of mammal, the body weight of the mammal, the composition of the aggregate, the value of the desired blood glucose level, the body condition and other factors. In short, the amount of insulin can vary according to actual needs.

Accordingly, the present invention provides a method for lowering blood glucose in a subject in need thereof, comprising orally administering to a subject in need thereof, for example, a patient with diabetes mellitus, the oral formulation of insulin.

In the present invention, the blood glucose of a human in need of lowering blood glucose, for example, a patient with diabetes mellitus, can be lowered by being administered orally with the oral formulation to achieve a therapeutic effect.

In some particular embodiments of the present invention, a therapeutically effective amount of insulin is 10-1000 U/ml of human recombinant insulin in an aggregate suspension.

In some particular embodiments of the present invention, the oral formulation of insulin takes about 15 minutes or less to enter the portal circulation.

In the present invention, the diabetes mellitus includes, but is not limited to, insulin-dependent reduced glucose tolerance, early diabetes, late diabetes, type I diabetes, type II diabetes, gestational diabetes, special types of diabetes, and the like.

The present invention provides a method for treating diabetes mellitus and reducing incidence rate of systemic hyperinsulinemia associated with long-term use of insulin.

In the present invention, the oral formulation of insulin may be taken at a time including, but not limited to, before a meal, after a meal, at night, and when there is a need to lower blood glucose.

In the present invention, a period of the administration of the oral formulation of insulin to a subject in need thereof may be determined based on the state of the disease progression. A chronic diabetic patient may be administered for at least two weeks or more. A gestational diabetic patient may be administered for the whole pregnancy period or when blood glucose is unstable. A patient requiring lifelong blood glucose control may be administered for a lifetime.

It should not be considered that the oral formulation used in the present invention is limited to the preferred embodiments described above. Other matrix materials, e.g., microporous particles, can be combined with the aggregate of insulin and chalcogen heterocyclic compound into an oral medicament for oral administration.

In the present invention, the oral formulation of insulin further comprises an additional therapeutic agent;

wherein the additional therapeutic agent is a subcutaneously-injectable medicament and/or an additional oral medicament for lowering blood glucose.

That is, the oral formulation of insulin of the present invention can be used in combination with a drug for lowering blood glucose to achieve a better glucose-lowering efficacy through combination therapy. The drug for lowering blood glucose can be administered by subcutaneous injection, including but not limited to, glucagon-like polypeptide-1, a glucagon-like polypeptide-1 receptor agonist (such as exenatide and liraglutide), and a glucose-dependent insulinotropic polypeptide; or can be administered orally, including, but not limited to a sulphonylurea, a glinide, a biguanide, an α-glucosidase inhibitor, a thiazolidinedione, and dipeptidyl peptidase-4.

In the present invention, the aggregate for the oral formulation of insulin or a protein-based drug can be prepared by any suitable method. Preferably, the aggregate is, preferably but not necessarily, prepared by a method of any embodiment herein.

In the present invention, a dry powder (e.g., a lyophilized powder) of the aggregate is preferably provided in combination with an enteric coated capsule as a composition, wherein the amount of insulin in the composition is a customized amount for the oral administration to a mammal. The size of the oral capsule is related to the species of mammal to which it is administered, as the size of the pharynx varies from mammal to mammal.

In another preferred aspect of the present invention, the composition is placed in a container. Preferably, the amount of insulin in the container is customized to be an amount for single or multiple oral administrations to a mammal. The container may be a container in compliance with the relevant regulations for pharmaceutical packaging materials, which can accommodate an orally administered capsule, such as a blister pack, a plastic bottle, a glass vial and/or other suitable containers, accompanied by instructions for use.

It should be mentioned that the composition for oral administration may also be a tablet formed by pressing a dry powder of the aggregate together with a pharmaceutically acceptable excipient, which is administered orally.

The present invention provides an oral formulation of insulin with a customized amount comprising the aggregate of insulin and chalcogen heterocyclic compound, wherein the amount of insulin in the aggregate is customized for oral administration of insulin to a human.

In some particular embodiments of the present invention, the oral formulation comprises an aqueous suspension of the aggregate formed by assembling insulin and chalcogen heterocyclic compound, wherein the amount of insulin is a therapeutically effective amount.

In some particular embodiments of the present invention, the oral formulation comprises a therapeutically effective amount of the aggregate formed by assembling insulin and chalcogen heterocyclic compound, a pharmaceutically acceptable coating and a capsule.

In some particular embodiments of the present invention, the oral formulation may be a tablet prepared from a therapeutically effective amount of the aggregate formed by assembling insulin and chalcogen heterocyclic compound and a pharmaceutically acceptable excipient.

In some particular embodiments of the present invention, the oral formulation is loaded in a container, wherein the amount of insulin is customized for a single or multiple oral administrations to a human.

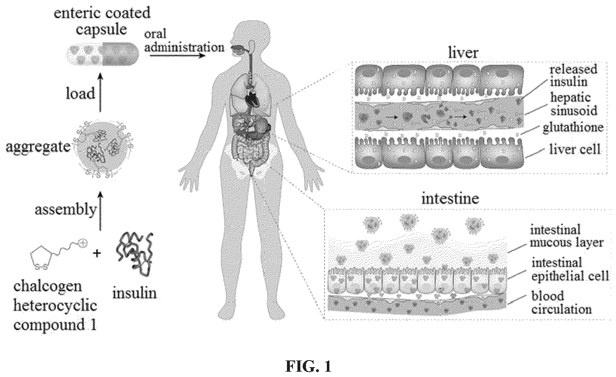

FIG. 1 is a schematic diagram showing a process of producing an oral formulation of insulin with chalcogen heterocyclic compound, loading in an enteric-coated capsule, and then performing oral administration for lowering blood glucose in the present invention. As shown in FIG. 1, in the present invention, for example, human recombinant insulin, is assembled with a chalcogen heterocyclic compound to form an aggregate. The aggregate is loaded into an enteric-coated capsule and administered orally. After entering the gastrointestinal tract, the aggregate enters into the cytoplasm directly through the thiol exchange route at small intestine, subsequently enters into the blood from the intestinal epithelial cells through the thiol exchange route again, and reaches to the hepatic sinusoids via the portal vein circulation. The molecular bond of polychalcogen in the aggregate is cleaved by glutathione in the hepatic sinusoids to release insulin to lower blood glucose.

Compared to the prior art, the present invention provides an aggregate, which is formed by assembling a chalcogen heterocyclic compound and insulin. The aggregate can be further prepared into an oral formulation of insulin. The oral formulation of insulin has the following beneficial effects:
(1) The oral formulation of insulin provided by the present invention is prepared by assembling insulin and a chalcogen heterocyclic compound to obtain an aggregate. Wherein the B group in the chalcogen heterocyclic compound represented by formula I has an intermolecular interaction with insulin, such that the molecules of chalcogen heterocyclic compound represented by formula I are in close proximity to each other, resulting in an increased local concentration, which facilitates ring-opening polymerization of the A group in the compound, allowing the compound to be presented in the form selected from the group consisting of a monomer, a short-chain oligomer, a long-chain polymer, and a combination thereof. The compound and insulin are ultimately assembled into a stable aggregate as a colloidal particle to achieve the encapsulation of insulin. This preparation process requires mild condition and simple operation, does not require additional functional additives, and has a low production cost.
(2) The oral formulation of insulin of the present invention is an aggregate formed by self-assembly of insulin with chalcogen heterocyclic compound. Compared to insulin encapsulated by liposome, an amphiphilic insulin micelle, and other oral formulations of insulin produced by other encapsulation methods, the aggregate assembled from insulin and chalcogen heterocyclic compound has a better stability in an acidic condition and a simulated gastrointestinal fluid, thus avoiding degradation by strong acidity in the stomach and digestive enzymes in gastrointestinal tract.
(3) After the oral formulation of insulin of the present invention enters the gastrointestinal tract of a mammal, a dynamic chemical exchange reaction between the molecular bond of polychalcogen on the surface of the aggregate and the sulfhydryl on the surface of mucoproteins and other glycoproteins promotes the rapid penetration of the aggregate through the intestinal mucus layer, wherein the aggregate of insulin and chalcogen heterocyclic compound does not destroy the tight junction protein between the epithelial cells. The aggregate of insulin and chalcogen heterocyclic compound, without destroying the tight junction protein between the epithelial cells, enters the cytoplasm via a non-endocytosis transcellular pathway by causing a dynamic chemical exchange reaction between the polychalcogen on the surface of the aggregate and the sulfhydryl on the outer surface of an epithelial cell membrane, avoiding being degraded by a lysosome. Then the aggregate enters into circulation system via intestinal epithelial cells by causing a dynamic chemical exchange reaction with the sulfhydryl on the inner surface of an epithelial cell membrane. The aggregate reaches the hepatic sinusoids via the portal circulation, and is depolymerized by glutathione in the hepatic sinusoids to release insulin to lower blood glucose. This process promotes the uptake of insulin by epithelial cells, effectively imitates the physiological transport route of insulin secreted by pancreas, and achieves a high bioavailability of insulin and a fast and long effect of lowering blood glucose by insulin, indicating that the oral formulation is useful in treating diabetes.
(4) The aggregate formed by self-assembly of insulin and chalcogen heterocyclic compound can be prepared, in flexible combination with various pharmaceutically acceptable excipients and formulation techniques, into an oral formulation in different forms such as enteric-coated capsules, a powder, a tablet, a granule, a suspension, and a dropping pill, which are convenient for patients with different medication habits.

Therefore, the oral formulation of insulin prepared with the chalcogen heterocyclic compound is more effective for use in the treatment of diabetes.

### BRIEF DESCRIPTION OF DRAWINGS

FIG. 1 is a schematic diagram showing a process of producing an oral formulation of insulin with chalcogen heterocyclic compound, loading in an enteric-coated capsule, and then performing oral administration for lowering blood glucose in the present invention.

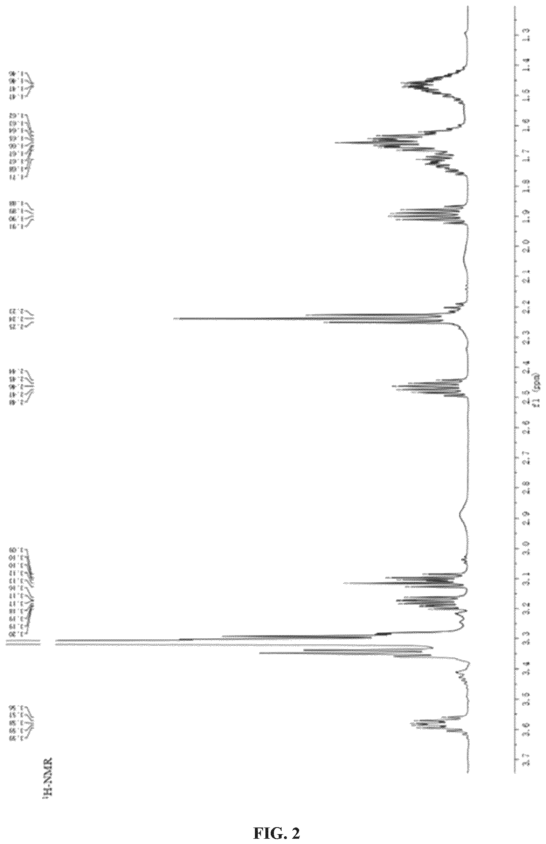

FIG. 2 shows the nuclear magnetic resonance spectrum of the chalcogen heterocyclic compound 1.

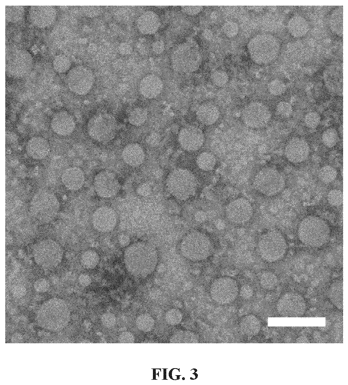

FIG. 3 shows the transmission electron microscope image of the aggregate 1 formed by assembling insulin and the chalcogen heterocyclic compound 1; scale bar: 100 nm.

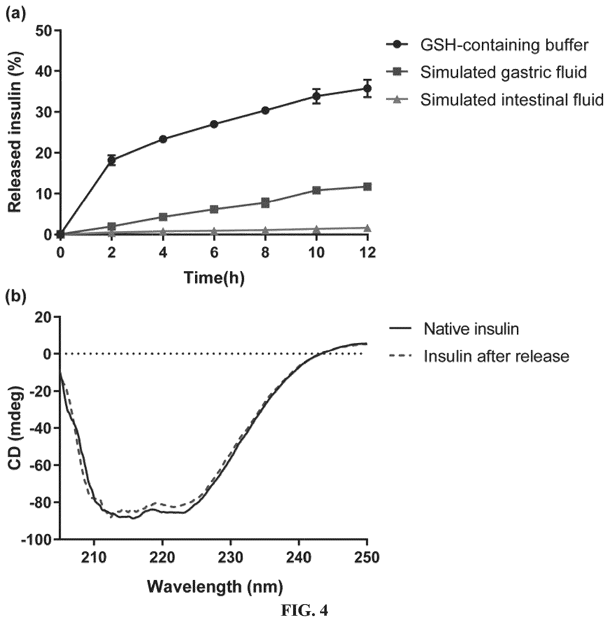

FIG. 4 (a) shows the kinetic curve of the release of insulin from the aggregate 1 formed by assembling insulin and the chalcogen heterocyclic compound 1 in a simulated gastrointestinal fluid and a buffer containing 7 mM glutathione; and FIG. 4 (b) shows the circular dichroism spectrum of insulin released from the aggregate 1.

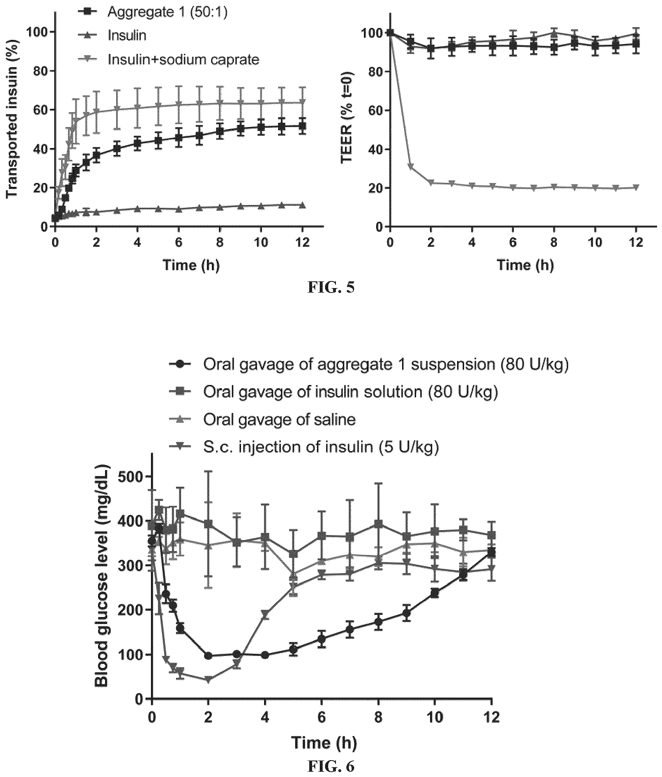

FIG. 5 (a) shows the changes over time in permeation efficiency of insulin transported by the aggregate 1 formed by assembling insulin and the chalcogen heterocyclic compound 1 through transcellular pathway in an *in vitro* intestinal tract model; and FIG. 5 (b) shows the changes over time in the transepithelial electrical resistance of the cell monolayer of the *in vitro* intestinal tract model after added with the aggregate 1, free insulin, and insulin with sodium caprate respectively.
FIG. 6 shows the changes over time in blood glucose level of diabetic model mice after administered orally with a suspension of the aggregate 1 formed by assembling insulin and the chalcogen heterocyclic compound 1, a solution of free insulin, and physiological saline respectively, and after injected subcutaneously with insulin only.

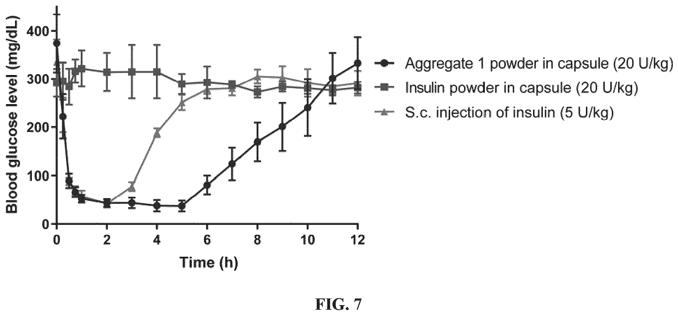

FIG. 7 shows the changes over time in blood glucose level of diabetic model mice after administered orally with an enteric-coated capsule loading a lyophilized powder of the aggregate 1 formed by assembling insulin and the chalcogen heterocyclic compound 1, and an enteric-coated capsule loading a lyophilized powder of free insulin respectively, and after injected subcutaneously with insulin only.

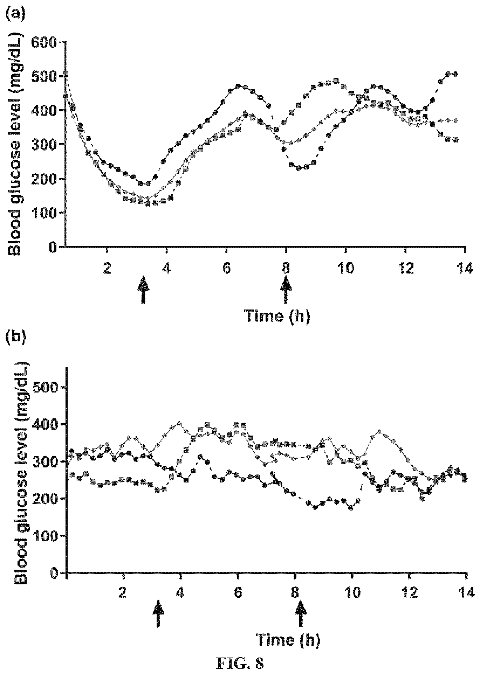

FIG. 8 shows the changes over time in blood glucose level of diabetic model pigs after administered orally with (a) an enteric-coated capsule loading a lyophilized powder of the aggregate 1 formed by assembling insulin and the chalcogen heterocyclic compound 1, and (b) an enteric-coated capsule loading a lyophilized powder of free insulin respectively, wherein the arrow indicates the time point when the diabetic model pigs were fed.

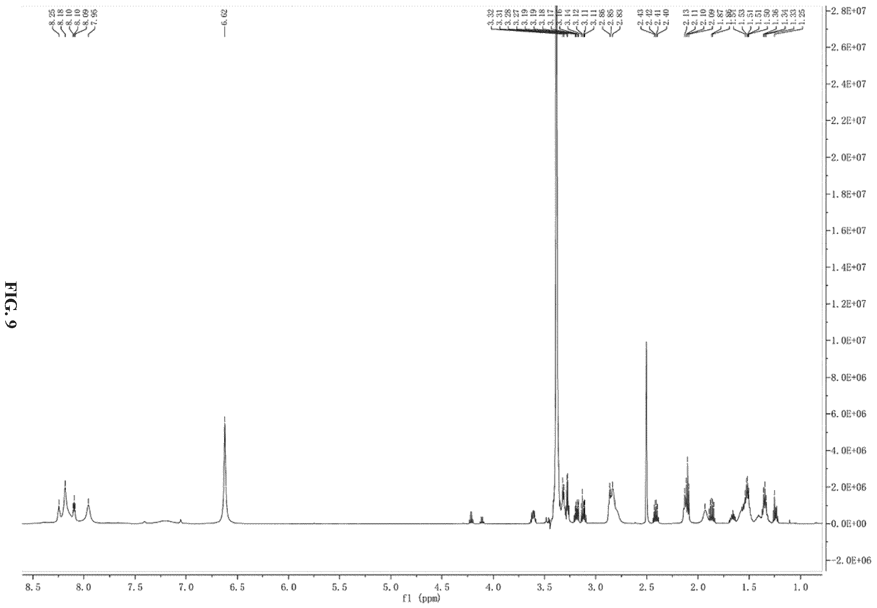

FIG. 9 shows the nuclear magnetic resonance spectrum of the chalcogen heterocyclic compound 4.

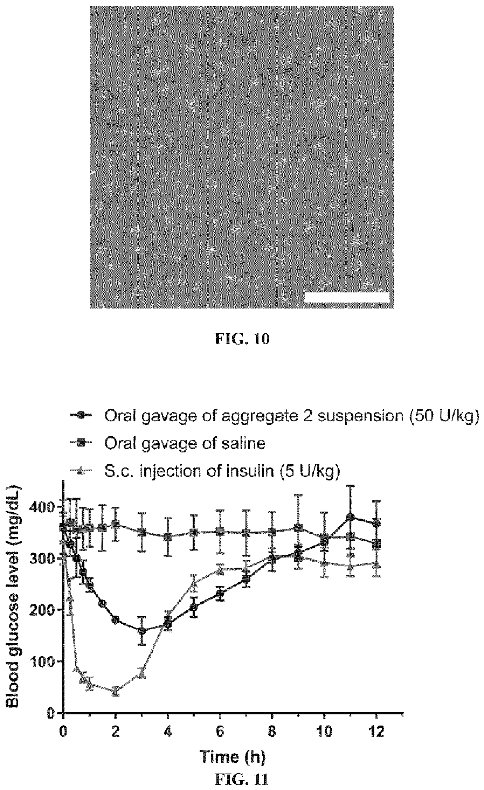

FIG. 10 shows the transmission electron microscope image of the aggregate 2 formed by assembling insulin and the chalcogen heterocyclic compound 4; scale bar: 100 nm.
FIG. 11 shows the changes over time in blood glucose level of diabetic model mice after administered orally with a suspension of the aggregate 2 formed by assembling insulin and the chalcogen heterocyclic compound 4, a solution of free insulin, and physiological saline respectively, and after injected subcutaneously with insulin only.

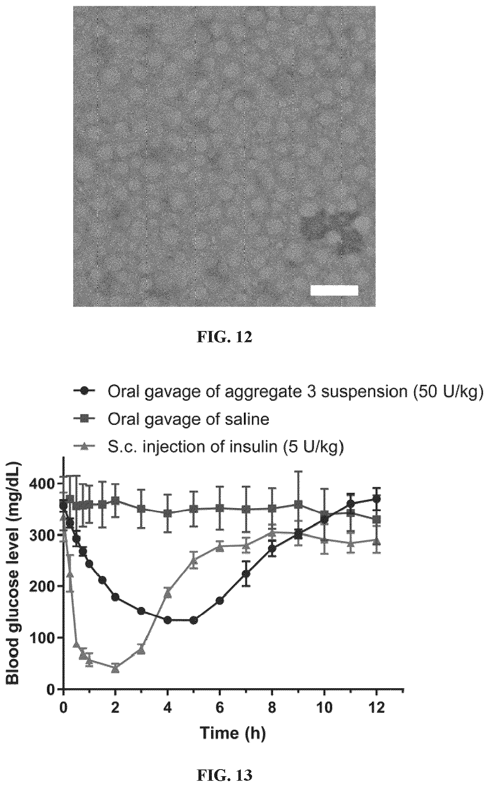

FIG. 12 shows the transmission electron microscope image of the aggregate 3 formed by assembling insulin and the chalcogen heterocyclic compound 10; scale bar: 100 nm.
FIG. 13 shows the changes over time in blood glucose level of diabetic model mice after administered orally with a suspension of the aggregate 3 formed by assembling insulin and the chalcogen heterocyclic compound 10, a solution of free insulin, and physiological saline respectively, and after injected subcutaneously with insulin only.

### DETAILED DESCRIPTION

In order to further illustrate the present invention, the aggregate formed by assembling a chalcogen heterocyclic compound and insulin of the present invention is described in detail below in conjunction with examples.

The experimental materials and related verification methods involved in the following specific examples are as follows.
(1) Chemical and biological reagents: The reaction reagents and organic solvents involved in the chemical synthesis of the chalcogen heterocyclic compound 1 in the example are commercially available. Ultrapure water was prepared by using Milli-Q water purification system (18.2 MΩ). Streptozotocin (STZ) was purchased from Shanghai Aladdin Biochemical Technology Company. Human recombinant insulin was purchased from Shanghai Yuanpei Biotechnology Co., Ltd. Intestinal mucoprotein of type III, derived from porcine stomach, was purchased from Sigma Aldrich. Transwell membrane insert chamber was purchased from Corning Company. Capsules for oral administration to mice and pigs were purchased from Torpac (USA). Enteric-coating EUDRAGIT L30 D-55 was purchased from Shanghai Changwei Pharmaceutical Excipients Company.
(2) Cell line and cell culture: Cell line is human colon cancer cells Caco-2, which was purchased from the Shanghai Cell Bank of the Chinese Academy of Sciences. Caco-2 cells were cultured in MEM medium (Corning) added with fetal bovine serum (Hyclone) at a final concentration of 20% and penicillin-streptomycin at a final concentration of 100 IU/mL in an atmosphere containing 5% CO₂ at 37°C.
(3) Mammals: C57BL/6J mice and Bama mini-pigs were purchased from Wu Laboratory Animal Company.

The insulin mentioned in the following examples was recombinant insulin, and a ratio of the human recombinant insulin to the compound in the aggregate refers to a ratio of the amount of substance.

### Example 1

Compound 1 represented by the following structure formula was used in example 1,

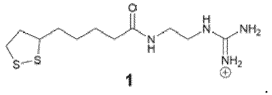

The compound 1 was synthesized as follows:

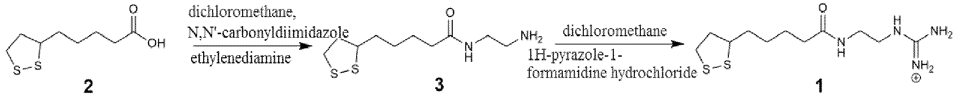

Firstly, compound 2 and N,N'-carbonyldiimidazole were dissolved in a solution of dichloromethane. The resulting solution was added dropwise to a solution of dichloromethane containing dichloromethane in an ice water bath, stirred for 40 minutes, and then stirred for 30 minutes at room temperature. After the reaction was completed, the obtained solution was washed with saline. Then the organic phase was dried over anhydrous sodium sulfate to remove water, and then concentrated under reduced pressure to obtain compound 3 as a yellow oil. Compound 3 was dissolved in dichloromethane, added with 1H-pyrazole-1-formamidine hydrochloride, stirred at room temperature for 4 hours, and distilled under reduced pressure to remove solvent. The residue was dissolved in methanol, added with ether for precipitation, and the resulting solid was collected and washed with ethyl ether to obtain the compound 1 in this example as a light yellow solid.

The structure of the resulting product was characterized by nuclear magnetic resonance. The result is shown in FIG. 2.

**Preparation of an oral formulation of insulin using the chalcogen heterocyclic compound 1 and characterization:** (1) A recombinant human insulin was mixed with the chalcogen heterocyclic compound 1 in a ratio of 1:50 by the amount of substance in a 20 mM Tris-HCl buffer solution with a pH of 7.0, and incubated for 30 minutes to obtain a suspension. (2) The suspension obtained in (1) was dialyzed or centrifuged to remove unreacted free insulin, free compound 1 and free oligomers to obtain an aggregate assembled from insulin and the chalcogen heterocyclic compound 1. Although not wishing to be limited by a particular theory, based on experimental studies, the inventors believe that in this process, an electrostatic or/and salt bridge interactions between the positively charged guanidine group of the compound 1 and the carboxyl of the insulin bring the molecules of the compound 1 into close proximity to increase the locally effective concentration of the compound 1, thereby facilitating the ring-opening polymerization of the chalcogen heterocyclic functional group to form oligomers, which were further self-assembled with the insulin to form an aggregate to achieve encapsulation of insulin.

As shown in FIG. 3, the aggregate produced in this example, as observed under transmission electron microscopy, had an average particle size of approximately 50 nm. The encapsulating efficiency of the insulin is about 70%.

### Study of kinetics of release of insulin from the aggregate of chalcogen heterocyclic compound and insulin under different conditions

(1) Aggregates labeled with FITC were dispersed in a simulated gastric fluid, a simulated intestinal fluid and a physiological buffer solution containing 7 mM glutathione for simulation of hepatic sinusoids respectively, and placed in a dialysis cup of a micro-dialysis device, wherein an isotonic solution was placed in the collection tube of the micro-dialysis device. (2) At the set time points, 50 µL of solution was collected from outside the dialysis cup and an equal amount of solution was replenished into the micro-dialysis device. (3) After 50 µL of the solution was diluted into 200 µL, fluorescence intensity of the solution was measured using a fluorescence spectrophotometer and the standard curve was established to determine the amount of insulin released.

As shown in FIG. 4a, almost no insulin was released from the aggregate during 12 hours of incubation in the simulated intestinal fluid; a small amount of insulin was released slowly in the simulated gastric fluid; and insulin could be released rapidly in the physiological buffer solution containing 7 mM glutathione. In addition, as shown in FIG. 4b, it was characterized by circular dichroism that the insulin released had an intact secondary structure, which is one of the hallmarks of bioactivity maintenance of insulin. Simulated gastric and simulated intestinal fluids are commonly used as models to study the stability of oral formulations after oral administration. The results of this experiment showed that the aggregate obtained in this example was stable in the gastrointestinal environment.

### Transcellular transport of the aggregate of insulin and chalcogen heterocyclic compound in intestinal model in vitro and integrity of epithelial cell layer

(1) Caco-2 cells were plated in a 24-well Transwell chamber and cultured for 21 days. The trans-epithelial electrical resistance (TEER) of the cell monolayer was greater than 300 Ω-cm² as detected by a Millicell electrical resistance system, indicating that tight junctions were formed between Caco-2 cells, and the intestinal epithelial cell monolayer model was successfully established *in vitro.* (2) The aggregate was added to the upper Transwell chamber at 20 µg/mL, the concentration of insulin in the liquid in the lower Transwell chamber was detected every 1 hour, and the TEER was monitored continuously for 12 hours.

Experimental results. As shown in FIG. 5a, the aggregate of human recombinant insulin and chalcogen heterocyclic compound transported insulin across cells with an efficiency of approximately 43%. However, there was almost no trans-epithelial cell transport of free human recombinant insulin in the control experiment.

As shown in FIG. 5b, the aggregate did not cause significant changes in TEER in this experiment, indicating that the aggregate does not disrupt intercellular tight junctions during transcellular transport, i.e., the aggregate does not transport via a paracellular pathway. To further verify the integrity of the epithelial cell layer, a permeation enhancer (sodium caprate) was added in the control experiment, and it was found that the TEER was drastically reduced, indicating that the integrity of the epithelial cell layer was disrupted.

### Change in blood glucose level of diabetic model mice after administered orally with the aggregate of insulin and chalcogen heterocyclic compound

(1) Streptozotocin (STZ) was intraperitoneally injected into 6-8 week-old C57BL/6J mice for five consecutive days to induce pancreatic islet B-cell damage. On the 17th day after the first injection, the blood glucose concentration of the mice was measured, and the diabetic mouse model was considered to be successfully established when the blood glucose concentration was higher than 300 mg/dL. (2) After the diabetic model mice were orally administered with a suspension of the above aggregate at an amount of insulin of 80 U/kg, blood was collected from the tail vein of the mice at different time points to monitor the change in blood glucose. In control experiment 1, an equal amount of free insulin was administered orally to the diabetic model mice. In control experiment 2, an equal volume of physiological saline was administered orally to the diabetic model mice. In control experiment 3, insulin was injected subcutaneously to the diabetic model mice at 5 U/kg.

Experimental results: As shown in FIG. 6, after the oral administration of the aggregate, the blood glucose concentration of diabetic model mice was significantly reduced by about 20% within 15 minutes and by about 30% after 60 minutes. The blood glucose concentration of the diabetic model mice was reduced by 66% over the entire period from 15-600 minutes. In control experiments 1 and 2, diabetic model mice administered with free insulin or physiological saline showed little reduction in blood glucose concentration. In control experiment 3, 15 minutes after the subcutaneous injection of insulin, the diabetic model mice showed significant reduction in blood glucose concentration, but this reduction was maintained for a short period of time, with a reduction of 30-50% within only 15-180 minutes.

These results showed that after the suspension of the aggregate entered the gastrointestinal tract, insulin was encapsulated by the chalcogen heterocyclic compound on the surface thereof, which can effectively avoid destruction by the strongly acidic environment and digestive enzymes in the gastrointestinal tract. The aggregate of insulin and chalcogen heterocyclic compound enters the cytoplasm via a non-endocytosis transcellular pathway by causing a dynamic chemical exchange reaction between the molecular bond of polychalcogen on the surface of the aggregate and the sulfhydryl on the outer surface of an epithelial cell membrane, avoiding being degraded by a lysosome. Then the aggregate enters into circulation system via intestinal epithelial cells by causing a dynamic chemical exchange reaction with the sulfhydryl on the inner surface of an epithelial cell membrane. The aggregate reaches the hepatic sinusoids via the portal circulation, and is depolymerized by glutathione in the hepatic sinusoids to release insulin. This process promotes the uptake of insulin by epithelial cells, and achieves a high bioavailability of insulin. According to this process, the oral formulation of insulin exerts a fast and long effect of lowering blood glucose.

Change in blood glucose level of diabetic model mice after orally administered with an enteric-coated capsule loading the aggregate of insulin and chalcogen heterocyclic compound
(1) The aggregate was prepared into a lyophilized powder and loaded in an enteric-coated capsule. In control experiment 1, an equal amount of free insulin was loaded in an enteric-coated capsule. (2) A diabetic mice model was established according to the method as above. The mice were administered with recombinant insulin at an amount of 20 U/kg, after which the blood was taken from the tail vein of the mice at different time points to monitor the change in blood glucose concentration. In control experiment 2, insulin was injected subcutaneously to the diabetic model mice at 5 U/kg.

Experimental results: As shown in FIG. 7, after orally administered with the aggregate, the diabetic model mice showed a significant reduction in blood glucose concentration by about 30% within 30 minutes and by about 45% after 60 minutes. The blood glucose concentration of the diabetic model mice was reduced by 75% over the entire period from 30-600 minutes. In control experiment 1, the diabetic model mice administered with the enteric-coated capsule loading free insulin showed little reduction in blood glucose concentration. In control experiment 2, 15 minutes after the subcutaneous injection of insulin, the diabetic model mice showed a significant reduction in blood glucose. However, this reduction was maintained for a short period of time, with a reduction of 30-50% within only 15-180 minutes.

### Change in blood glucose level of diabetic model pigs after orally administered with an enteric-coated capsule loading the aggregate of insulin and chalcogen heterocyclic compound

(1) Streptozotocin (STZ, with a concentration of 75 mg/mL) was infused intravenously to 4-month-old Bama mini-pigs at 150 mg/kg to induce pancreatic islet B-cell damage. On the 7th day after the first infusion, the blood glucose concentration of the pigs was measured, and the diabetic pig model was considered to be successfully established when the blood glucose concentration was higher than 300 mg/dL. (2) The aggregate was prepared into a lyophilized powder and loaded in an enteric-coated capsule. In control experiment 1, an equal amount of free insulin was loaded in an enteric-coated capsule. (3) The diabetic model pigs were administered with recombinant insulin at an amount of 20 U/kg, after which the blood was taken from the jugular vein of the pigs at different time points to monitor the change in blood glucose concentration. (4) The diabetic model pigs were given normal food at the 3rd and 8th hour after administration, and the food bowl was removed after 15 minutes.

Experimental results: As shown in FIG. 8, the blood glucose concentration of the diabetic model pig was significantly reduced by about 7% within 30 minutes and by about 35% after 60 minutes. The blood glucose concentration of the diabetic model pig was reduced over the entire period from 30-840 minutes. For both meals during the experiment, the blood glucose concentration increased after the meal, and reduced subsequently, wherein the blood glucose concentration was reduced by 55-60%. In control experiment 1, the diabetic model pigs administered with the enteric-coated capsule loading free insulin showed little reduction in blood glucose concentration.

### Example 2

Compound 4 represented by the following structure formula was used in example 2

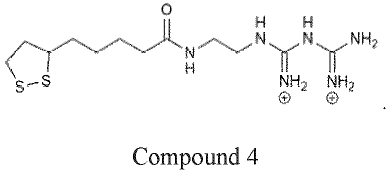

The compound 4 in the present invention was synthesized as follows:

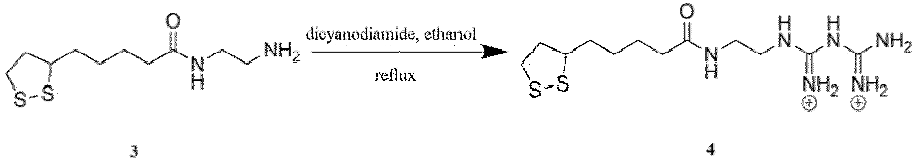

Compound 4 was prepared from compound 3. Hydrochloric acid was mixed with anhydrous ethanol to obtain a mixture. The mixture was stirred evenly, added with compound 3, and added with an equal molar equivalent of dicyandiamide. The resulting mixture was heated to reflux for 12 hours under constant stirring, and then subjected to a column to obtain compound 4.

The structure of the resulting product was characterized by nuclear magnetic resonance, and the result is shown in FIG. 9.

**Preparation of an oral formulation of insulin using the chalcogen heterocyclic compound 4 and characterization:** (1) Recombinant human insulin was mixed with the chalcogen heterocyclic compound 4 in a ratio of 1: 50 by the amount of substance, and incubated in a 20 mM Tris-HCl buffer solution with a pH of 7.0 for 10 minutes. (2) The suspension obtained in (1) was dialyzed or centrifuged to remove unreacted free insulin, free compound 4, and free oligomers, to obtain aggregate 2 formed by assembling insulin and the chalcogen heterocyclic compound 4.

As shown in FIG. 10, the aggregate 2 produced in this example, as observed under transmission electron microscopy, had a particle size of 30-50 nm. The encapsulating efficiency of the insulin is about 70%.

**Change in blood glucose level in diabetic model mice after administration of a suspension of the aggregate of insulin and chalcogen heterocyclic compound:** The diabetic model mice were orally administered with a suspension of the aggregate 2 obtained above at an amount of insulin of 50 U/kg, and then the blood was collected from the tail vein of the mice at different time points to monitor the change in blood glucose concentration. In control experiment 1, an equal volume of physiological saline was administered orally to the diabetic model mice, and in control experiment 2, insulin was injected subcutaneously to the diabetic model mice at 5 U/kg.

Experimental results: As shown in FIG. 11, after orally administered with the aggregate 2, the diabetic model mice showed a significant reduction in the blood glucose concentration by about 20% within 30 minutes and by about 30% after 60 minutes. The blood glucose concentration of the diabetic model mice was reduced by 50% over the entire period from 15-600 minutes. In control experiment 1, the diabetic model mice administered with physiological saline showed little reduction in the blood glucose concentration. In control experiment 2, 15 minutes after the subcutaneous injection of insulin, the diabetic model mice showed a significant reduction in blood glucose concentration. However, this reduction was maintained for a short period of time, with a reduction of 30-50% within only 15-180 minutes.

### Example 3

Compound 10 represented by the following structure formula was used in example 3

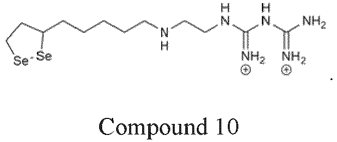

The compound 10 in the present invention was synthesized as follows:

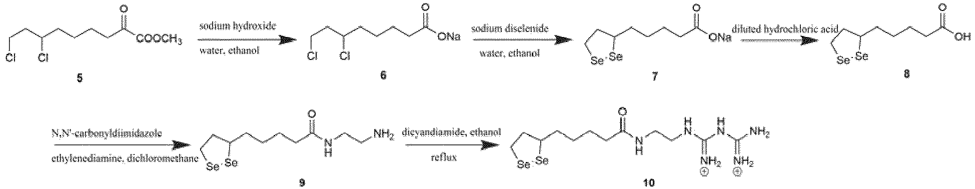

Seleno-octanoic acid 8 was first prepared. Compound 5 was added to a round bottom flask, then added with anhydrous ethanol, and 2 times molar equivalent of sodium hydroxide and water sequentially for a reaction at 50°C for 2 hours to obtain compound 6. Then an aqueous sodium diselenide solution was added dropwise to the round bottom by a syringe pump within 2 hours at 70°C. After the addition was completed, the reaction was performed at 70°C for 3 hours, to obtain compound 7. Then the mixture was cooled to 40°C, added with activated carbon, stirred for 30 minutes, and filtered while still hot to remove unreacted impurities and activated carbon. The obtained filtrate was placed on an ice-water mixture, cooled to 0°C, and added with 5% dilute hydrochloric acid dropwise under rapid stirring to adjust the pH to 2. A large number of brown solids were precipitated, subjected to suction filtration, and dried, to obtain compound 8 (selenooctanoic acid).

Compound 8 and N,N'-carbonyldiimidazole were dissolved in dichloromethane solution to obtain a mixture. Then the mixture was added dropwise to a dichloromethane solution containing ethylenediamine placed in an ice-water bath, stirred for 40 minutes, and then stirred at room temperature for 30 minutes. After the reaction was completed, the solution was washed with brine. The organic phase was dried over anhydrous sodium sulfate to remove water, and then concentrated under reduced pressure to obtain compound 9 as a yellow oil. Subsequently, hydrochloric acid was mixed with anhydrous ethanol to obtain a mixed solution, which was stirred evenly. The mixed solution was added with compound 9 and an equal molar equivalent of dicyandiamide, heated to reflux for 12 hours with constant stirring, and then subjected to a column to obtain compound 10.

The structure of the resulting product was verified by nuclear magnetic resonance spectroscopy.

**Preparation of an oral formulation of insulin using the chalcogen heterocyclic compound 10 and characterization:** (1) Recombinant human insulin was mixed with the chalcogen heterocyclic compound 10 in a ratio of 1:50 by the amount of substance, and incubated in a 20 mM Tris-HCl buffer solution with a pH of 7.0 for 10 minutes. (2) The suspension obtained in (1) was dialyzed or centrifuged to remove unreacted free insulin, free compound 10, and free oligomers, to obtain the aggregate 3 formed by assembling insulin and the chalcogen heterocyclic compound 10.

As shown in FIG. 12, the aggregate 3 produced in this example, as observed under transmission electron microscopy, had a particle size of approximately 50 nm. The encapsulating efficiency of insulin is about 65%.

**Change in blood glucose level in diabetic model mice after administration of a suspension of the aggregate of insulin and chalcogen heterocyclic compound:** The diabetic model mice were orally administered with a suspension of the above aggregate 3 at an amount of insulin of 50 U/kg. The blood was collected from the tail vein of the mice at different time points to monitor the change in blood glucose concentration. In control experiment 1, an equal volume of physiological saline was administered orally to the diabetic model mice, and in control experiment 2, insulin was injected subcutaneously to the diabetic model mice at 5 U/kg.

Experimental results: As shown in FIG. 13, after orally administered with the aggregate 3, the diabetic model mice showed a significant reduction in the blood glucose concentration by about 20% within 30 minutes and by about 30% after 60 minutes. The blood glucose concentration of the diabetic model mice was reduced by 55% over the entire period from 15-600 minutes. In control experiment 1, the diabetic model mice administered with physiological saline showed little reduction in the blood glucose concentration. In control experiment 2, 15 minutes after the subcutaneous injection of insulin, the diabetic model mice showed a significant reduction in blood glucose concentration. However, this reduction was maintained for a short period of time, with a reduction of 30-50% within only 15-180 minutes.

Since mammals such as mice and pigs are common models for human beings in drug testing, it can be demonstrated that a composition comprising an aggregate of insulin and chalcogen heterocyclic compound, such as a suspension, an enteric-coated capsule, a powder, a tablet, a granule, and a pill, could effectively lower the blood glucose level in human by oral administration, and could be particular useful in the treatment of diabetic patients.

Therefore, the production of an oral formulation of insulin using a chalcogen heterocyclic compound is simple in operation and low in cost. Moreover, the resulting oral formulation improves the pharmacokinetic and pharmacodynamic characteristics of insulin administered orally to achieve a high bioavailability, has a good effect on lowering blood glucose, and has a broad prospect for treating diabetes.

The above examples are described only to help understand the method and core concept of the present invention. It should be noted that, several improvements and modifications to the present invention may be made by those skilled in the art without departing from the principle of the present invention, and these improvements and modifications shall fall within the protection scope of the claims of the present invention.

## Claims

1. An aggregate formed by assembling a chalcogen heterocyclic compound and insulin; wherein the chalcogen heterocyclic compound has a structure represented by formula I:
A-L-B Formula I;
wherein, A is a heterocyclic group containing two or more same and/or different atoms of chalcogen selected from the group consisting of sulfur, selenium and tellurium;
B is a group interacting with an insulin molecule, and
L is a linker linking A and B.

2. The aggregate according to claim 1, wherein A has a structure selected from the group consisting of: 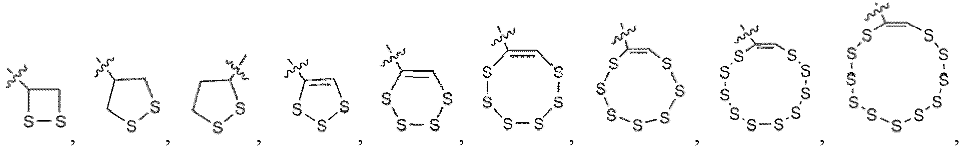 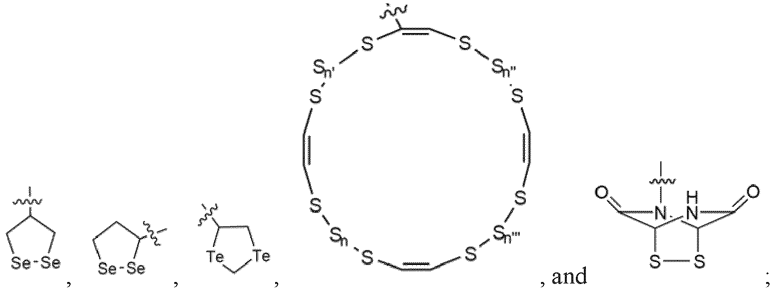
L is selected from the group consisting of a carbon-carbon bond, a carbon-boron bond, a carbon-nitrogen bond, a carbon-phosphorus bond, a carbon-oxygen bond, a carbon-sulfur bond, a carbon-selenium bond, a carbon-tellurium bond, a metal-ligand bond, a boron-ester bond, a disulfide bond, a ring-forming group, a hydrogen bond, a cleavable chemical bond, a supramolecular host-guest interaction, and a ligand-receptor recognition interaction; and
B is selected from the group consisting of a chemical/biological molecule recognizing insulin, a DNA complementary strand, an aptamer, a polypeptide having a coiled coil/zipper structure/superstructure, and a chemical group selected from the group consisting of: 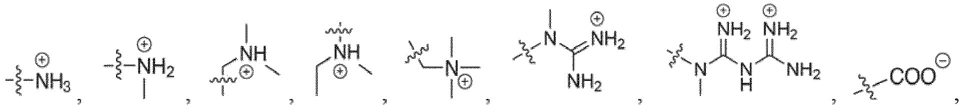 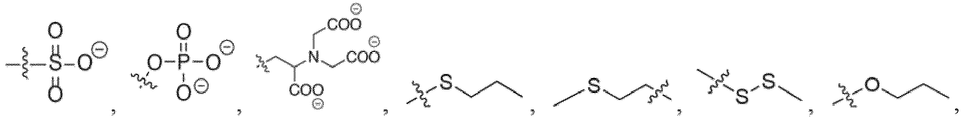 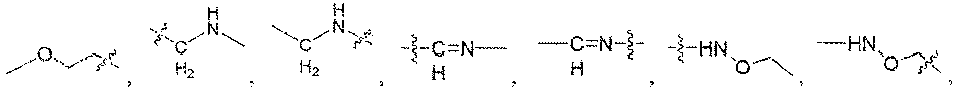 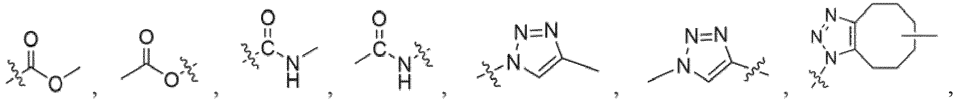 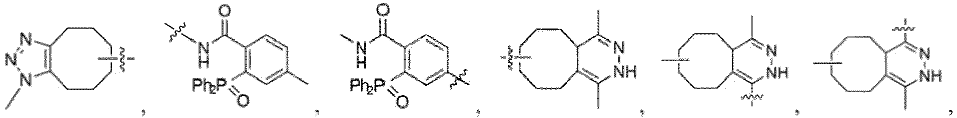 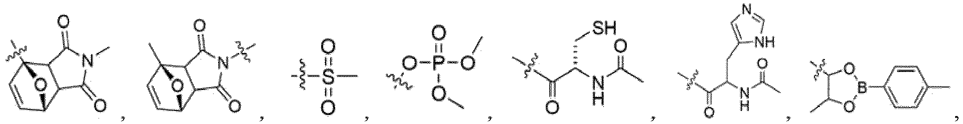 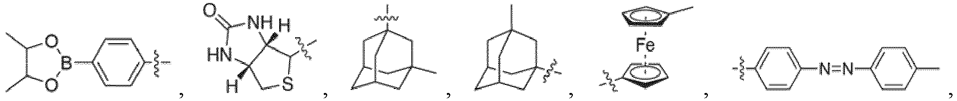 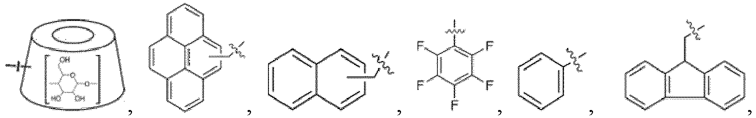 and a combination thereof.

3. The aggregate according to claim 1, wherein the chalcogen heterocyclic compound has a structure represented by formula II: 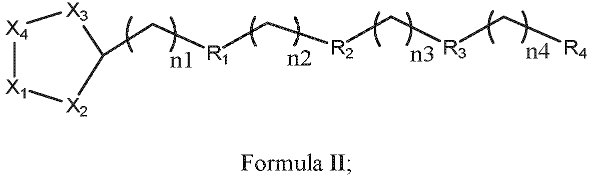
wherein, X₁, X₂, X₃, and X₄ are independently selected from the group consisting of S, Se, Te and C, and at least two of X₁, X₂, X₃, and X₄ are independently selected from the group consisting of S, Se and Te;
R₁, R₂, and R₃ are independently selected from the group consisting of a carbon atom, amido and imino;
R₄ is selected from carboxyl, amino, 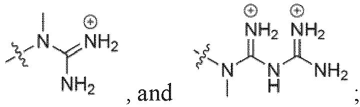 and
n1, n2, n3, and n4 are any integers independently selected from 1 to 6.

4. The aggregate according to claim 1, wherein the chalcogen heterocyclic compound has a structure selected from the group consisting of 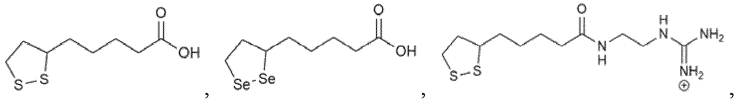 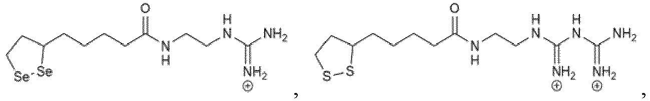 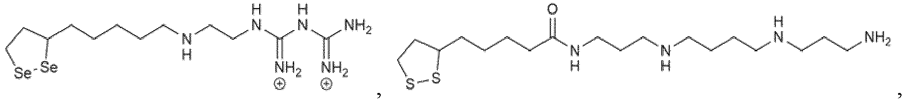 and a combination thereof.

5. The aggregate according to claim 1, wherein the insulin is selected from the group consisting of unmodified insulin, an insulin analogue, modified insulin, and a combination thereof.

6. The aggregate according to claim 1, wherein the unmodified insulin is selected from the group consisting of human insulin, human recombinant insulin, bovine insulin, porcine insulin, semi-synthetic insulin, biosynthetic insulin, and a combination thereof;
the insulin analogue is selected from the group consisting of insulin lispro, insulin glulisine, insulin aspart, insulin detemir, insulin glargine, insulin degludec; and a combination thereof; and
the modified insulin is selected from the group consisting of chemically modified insulin, biologically modified insulin, genetically engineered insulin, and a combination thereof.

7. The aggregate according to claim 1, wherein a molar ratio of the chalcogen heterocyclic compound to the insulin is 1-200: 1.

8. The aggregate according to claim 1, wherein the aggregate has a particle size of 10-500 nm.

9. A method for producing the aggregate according to any one of claims 1 to 8, comprising S1) incubating a solution of insulin with a solution of a chalcogen heterocyclic compound, assembling, and then obtaining an aggregate.

10. Use of the aggregate according to any one of claims 1 to 8 in the manufacture of a medicament for lowering blood glucose.

11. An oral formulation of insulin, comprising the aggregate according to any one of claims 1 to 8, and a pharmaceutically acceptable excipient.

12. The oral formulation of insulin according to claim 11, wherein the oral formulation is selected from the group consisting of an enteric-coated capsule, a powder, a tablet, a granule, a suspension, a drop pill, and a combination thereof.

13. The oral formulation of insulin according to claim 11, further comprising an additional therapeutic agent;
wherein the additional therapeutic agent is a subcutaneously-injectable medicament and/or an additional oral medicament for lowering blood glucose.

14. The oral formulation of insulin according to claim 13, wherein the additional therapeutic agent is selected from the group consisting of glucagon-like peptide-1, a glucagon-like peptide-1 receptor agonist, a glucose-dependent insulinotropic polypeptide, a sulphonylurea, a glinide, a biguanide, an α-glucosidase inhibitor, a thiazolidinedione, dipeptidyl peptidase-4, and a combination thereof.
